# EUROPEAN PATENT APPLICATION

(11) **EP 2 810 691 A1**
(43) Date of publication of application: **10.12.2014**
(21) Application number: 13170859.6
(22) Date of filing: 06.06.2013
(51) Int. Cl.: A61N 1/36, A61N 1/372, G06F 19/00

(54) **A system for planning and/or providing a therapy for neural applications**

(71) Applicant: Sapiens Steering Brain Stimulation B.V., 5656 AE Eindhoven (NL)
(72) Inventor: Martens, Hubert Cécile François, 5611 ND Eindhoven (NL); van Elswijk, Gijs Antonius Franciscus, 5644 NC Eindhoven (NL)
(74) Representative: Prinz & Partner mbB

(57) **Abstract**

The present invention relates to a system (10) for planning and/or providing a therapy for neural applications, especially for neurostimulation and/or neurorecording applications, comprising and/oder being connected and/or being connectable to at least one therapy delivery means (300) being capable to influence a volume of tissue of a patient (1), further comprising a steering means (400) which is configured such that at least the shape of the volume of tissue (TV) that shall be influenced by the at least one therapy delivery means (300) may be modelled and the necessary settings and/or implantation conditions of therapy delivery means (300) may be set accordingly. Further, the present invention relates to a system for neural applications (100), especially a system for neurostimulation and/or neurorecording applications (100), for instance a deep brain stimulation system (100).

## Description

The present invention relates to a system for planning and/or providing a therapy for neural applications, especially neurostimulation and/or neurorecording applications and a system for neural applications, especially a system for neurostimulation and/or neurorecording applications, for instance a deep brain stimulation system.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a stylet material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, the DBS lead has e.g. four 1.5 mm-wide cylindrical electrodes at the distal end spaced by 0.5 mm or 1.5 mm. The diameter of the lead is 1.27 mm and the metal used for the electrodes and the interconnect wires is an alloy of platinum and iridium. The coiled interconnect wires are insulated individually by fluoropolymer coating and protected in an 80A urethane tubing. With such electrode design, the current distribution emanates uniformly around the circumference of the electrode, which leads to stimulation of all areas surrounding the electrode.

The lack of fine spatial control over field distributions implies that stimulation easily spreads into adjacent structures inducing adverse side-effects in about 30% of the patients. To overcome this problem, systems with high density electrode arrays are being developed, hence providing the ability to steer the stimulation field to the appropriate target.

The clinical benefit of DBS is largely dependent on the spatial distribution of the stimulation field in relation to brain anatomy. To maximize therapeutic benefits while avoiding unwanted side-effects, precise control over the stimulation field is essential.

During stimulation with existing DBS leads there is an option to use monopolar, bipolar, or even tripolar stimulation. Neurostimulator devices with steering brain stimulation capabilities can have a large number of electrode contacts (n > 10) to which electrical settings such as current sources or grounding can be applied. Stimulation may be considered monopolar when the distance between the anode and cathode is several times larger than the distance of the cathode to the stimulation target. During monopolar stimulation in homogeneous tissue the electric field is distributed roughly spherical similar to the field from a point source. When the anode is located close to the cathode the distribution of the field becomes more directed in the anode-cathode direction. As a result the field gets denser and neurons are more likely to be activated in this area due to a higher field gradient.

The mechanisms of DBS are unknown. However, it is hypothesized that polarization (de- and/or hyperpolarization) of neural tissue is likely to play a prominent role for both suppression of clinical symptoms, as well as induction of stimulation-induced side-effects. In order to activate a neuron it has to be depolarized. Neurons are depolarized more easily close to the cathode than by the anode (about 3-7 times more depending on type of neuron, etc.).

Therefore, compared to monopolar stimulation the effect of bipolar stimulation is less spread of the electric field, a denser electric field between the anode and cathode, and more activated neurons close to the cathode. Bipolar stimulation is therefore used to focus the field to certain areas in cases when beneficial stimulation is not obtained during monopolar stimulation.

DBS leads are typically implanted via a stereotactic neurosurgical procedure. The planning of a stereotactic procedure involves to the identification of the DBS target (e.g. the subthalamic nucleus) on the basis of the MR or CT images of the patient's head/brain and defining a point within the target nucleus. Eventually the stereotactic planning station (e.g. a computer system) provides the stereotactic coordinates of the target point. The stereotactic coordinates can be referenced externally and thus be used in the operating room to precisely navigate the DBS lead to the selected point in the brain.

DBS exerts its therapeutic effects through electric stimulation of a volume of tissue surrounding the active electrode(s). In existing planning stations the DBS target is represented/defined as a point. The disadvantage of defining the DBS target as a point is that it does not accurately reflect the characteristics of the therapeutic intervention.

In fact, the DBS lead may be regarded as the conduit to provide what is actually more relevant for therapy, namely the electric field delivered to a certain brain region. Taking the stimulation volume into account during target planning becomes even more important when future DBS systems will be able to produce complex non-uniform field shapes. Moreover, distribution of electric potential in brain tissue may depend on conductivity characteristics of the respective tissue. Similar problems arise also in connection with drug delivery systems, systems for delivery of ultrasound energy like an array of transducers for delivery of ultrasound energy, light-emitting systems like an array of light-conductors, in particular e.g. waveguides.

It is therefore an object of the present invention to improve a system for planning and/or providing a therapy for neural applications and a system for neural applications, in particular in that the effect of a treatment may be more predictable a-priori and that the optimal treatment setting may be identified.

The above object is solved according to the present invention by a system for planning and/or providing a therapy for neural applications according to claim 1. Accordingly, a system for planning and/or providing a therapy for neural applications is provided, especially for neurostimulation and/or neurorecording applications, comprising at least one therapy delivery means being capable to influence a volume of tissue of a patient, further comprising a steering and/or field shaping means which is configured such that at least the shape of the volume of tissue that shall be influenced by the at least one therapy delivery means may be modelled and the necessary settings and/or implantation conditions of therapy delivery means may be set accordingly.

By this the advantage is achieved that the effect of a treatment may be more predictable a-priori and that the optimal treatment setting may be identified. So, the system may identify a therapy delivery means that is able to generate electrical field shapes that match the shape or shapes of the volume of tissue that shall be influenced by the at least one therapy delivery means of the target volume indicated by the user. The ability to take the steering and/or field shaping capabilities of the therapy delivery means systems into account during the surgical planning will allow optimizing the therapy/side effect balance of the treatment already prior to of e.g. an implantation of the therapy delivery means.

Additionally, it is possible that the system comprises at least one processing means being capable to combine specific anatomical data and/or functional data and information about the volume of tissue of a patient that can be influenced by the at least one therapy delivery means, wherein the at least one steering and/or field shaping means is configured such that the therapy delivered by the therapy delivery means may be steered depending on at least one output of the processing means.

Moreover, it is possible that the therapy delivery means is or comprises a drug delivery system and/or a system for delivery of ultrasound energy like an array of transducers for delivery of ultrasound energy and/or a light-emitting system like an array of light-conductors, in particular e.g. waveguides.

For instance, the therapy delivery means is or comprises a lead for neural applications, especially for neurostimulation and/or neurorecording applications.

The lead may comprise a plurality of electrodes, wherein the lead is capable to provide and/or generate at least one stimulation field.

Especially, the lead may be a lead for neural applications, preferably a lead for a neurostimulation and/or neurorecording system. Such a neurostimulation and/or neurorecording system may be e.g. a DBS system.

The lead may e.g. comprise at least one thin film, wherein the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or substantially the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

There may be an Advanced Lead Can element, which may comprise electronic means to address the plurality of electrodes and at least one Advanced Lead Can connecting means. Further, the Advanced Lead Can element may be hermetically or substantially hermetically sealed and may comprise electronic means to address the plurality of electrodes on the distal end of the thin film, which is arranged at the distal end and next to the distal tip of the lead. The plurality of electrodes may comprise more than 5-10 electrodes, e.g. 16 or 32 electrodes or in preferred embodiments e.g. 40 electrodes or more. The electrodes may be arranged such that the electrodes are substantially evenly distributed arranged all over the distal end of the lead.

Furthermore, it is possible that the plurality of electrodes forms a complex geometrical array and/or that the plurality of electrodes is arranged circumferentially around at least one section of the lead, especially around a section next to the distal tip end of the lead.

By means of a complex array a stimulation field of any desired shape may be formed and may be adjusted according to the patient's needs. So, a suitable and accurate tailor-made stimulation field may be provided.

A complex array may be formed by a plurality of electrodes, which are arranged circumferentially around a section next to the distal tip end of the lead. The electrodes may be e.g. arranged non-planar and non-coaxial and likewise a leopard pattern and may form a regular array. Several electrodes may form at one level a ring around the lead and the next ring may be slightly displaced such that e.g. one electrode of the second ring is partially arranged within the gap between to electrodes of the first ring. So, there are rings of electrodes in radial direction of the lead and columns of electrodes in axial direction of the lead.

Furthermore, it is possible that the system comprises a visualization means, wherein the visualization means is capable to visualize volume of tissue of a patient to be addressed by the therapy delivery means, especially in the context of the pre-operative and/or peri-operative images of the patient.

The visualization means may be or may comprise e.g. display. Advantageously, the visualization means may be or may comprise e.g. a touch screen.

So, one advantage of this system is that the physician will be supported to predict the effect of stimulation a-priori more precisely and based thereupon may select an optimal DBS lead for the given patient, or the system may identify a DBS lead that is able to generate electrical field shapes that match the shape of the target volume indicated by the physician or any other user. The ability to take steering capabilities of DBS systems into account during the surgical planning will allow optimizing the therapy/side effect balance of DBS already prior to of lead implantation.

Additionally, it is possible that the visualization means comprises input means and output means and is configured such that patient images may be loaded and/or displayed by means of the output means and that by means of the input means a target volume of tissue of a patient may be indicated, especially via voxel painting and/or by free-form drawing of 3D volumes on the patient images.

In case that the visualization means is a touch screen the touch screen provides both the input means and the output means.

Patient images may be e.g. X-Ray images, computer tomography (CT) images or magneto resonance images (MRI) or the like and the visualization means may comprise e.g. a loading module or at least one connection which is capable to load and/or unload patient images, in particular patient images of interest like in the field of DBS patient images of the brain.

The system may comprise an identifying means which is configured such that from the indicated target volume of tissue of a patient the most optimal settings and/or implantation conditions of therapy delivery means may be identified and especially set accordingly by the steering means.

In particular, it is possible that patient images are loaded and user can indicate a target volume, e.g. by voxel painting or by free-form drawing of 3D volumes on the patient images. The software identifies from the indicated target volume (TV), i.e. the volume of tissue that shall be influenced by the at least one therapy delivery means and/or a most optimal lead that generates field-shapes overlapping with the user indicated TV. The selected lead is positioned automatically by the system in an orientation that matches the TV, or it can be placed manually by the user on the images and TV.

Further, it is possible that the visualization means is configured such that at least one or more effective shape of the volume of tissue may be displayed and that the system comprises a simulation means which is capable to provide a feed-forward simulation of the shape of the volume of tissue that shall be provided by the at least one therapy delivery means and that visualization means is configured such that patient images may be combined with a feed-forward simulation of the shape of the volume of tissue that shall be provided by the at least one therapy delivery means.

For instance, in the field of DBS, patient images (e.g. MRI/CT) may be combined with feed-forward simulation of electrical field(s) that can be produced by the to-be-implanted DBS system. The user may select a DBS lead, and for this lead several "effective" volumes can be displayed (non-polarized electric field, directionally dependent electric field, neuronal activation). Volumes can be displayed in 2D by colour coding of MRI voxels (e.g. spread of electric field indicated by red voxels), or using 3D meshes. The user can position the lead and its effective volumes in such a way that leads to an optimal result, i.e. good overlap with the TV.

Furthermore, it is possible that the simulation means is configured such that information about tissue anisotropy and/or tissue inhomogenity is used for the simulation when providing a feed-forward simulation of the shape of the volume of tissue that shall be provided by the at least one therapy delivery means.

By this, the advantage is achieved that the provided feed-forward simulation of the shape of the volume of tissue that shall be provided by the at least one therapy delivery means is more accurate. For instance, information about tissue anisotropy/inhomogeneity (deduced from e.g. MRI/diffusion tensor imaging (DTI)) may be taken into account in the simulation of the electric field model.

Moreover, it is possible that the visualization means is configured such that the user may identify a target area and may outline a desired shape of the volume of tissue (TV) that shall be influenced by the at least one therapy delivery means, wherein especially the desired shape of the volume of tissue (TV) that shall be influenced by the at least one therapy delivery means is a desired electrical field coverage volume.

For instance, the user may identify a target area and may outline desired electrical field coverage volume. The system may compute and identify the optimal lead for this and may then show the lead and stimulation capabilities on top of this.

Furthermore, it is possible that the steering and/or field shaping means is configured such that at least one boundary-condition may be input, wherein especially the boundary-condition may be input via the input means.

Additionally, it is possible that the boundary condition is a certain range of lead-implantation angles and/or a lead position relative to the target volume (e.g. lateral to the and/or a certain area to remain unaffected by stimulation).

For instance, it is possible to allow the user to provide more boundary-conditions, e.g. by demanding a certain range of lead-implantation angles, by demanding a lead position relative to the target volume (TV) (e.g. lateral to the TV), by demanding certain areas to remain unaffected by stimulation, etc. Essentially, that will narrow the search-space for lead types and may narrow the orientation/location of the lead with respect to the target volume.

Moreover, it is possible that the steering and/or field shaping means is configured such that at least the shape of the volume of tissue that shall be influenced by the at least one therapy delivery means may be modelled and the necessary settings and/or implantation conditions of therapy delivery means may be set accordingly semi-automatically and/or automatically.

By an automatically and/or semi-automatically setting the advantage is achieved that the setting of the necessary settings and/or implantation conditions of therapy delivery means can be done very easy and more intuitively. A semiautomatic setting may be realized requiring a specific user input.

Moreover, the present invention relates to a system for neural applications with the features of claim 15. Accordingly, a system for neural applications is provided, especially a system for neurostimulation and/or neurorecording applications, for instance a deep brain stimulation system. The system for neural applications comprises at least one system for planning and/or providing a therapy for neural applications according to one of claims 1 to 14.

Furthermore, the following method for planning and/or providing a therapy for neural applications, especially a neurostimulation and/or neurorecording applications with the following features is explicitly disclosed:

Accordingly, the method for planning and/or providing a therapy for neural applications, especially a neurostimulation and/or neurorecording applications comprises at least the step of modeling at least the shape of the volume of tissue that shall be influenced by the at least one therapy delivery means and setting the necessary settings and/or implantation conditions of therapy delivery means accordingly.

Further, specific anatomical data and/or functional data and information about the volume of tissue of a patient that can be influenced by the at least one therapy delivery means may be combined and the therapy delivered by the therapy delivery means may be steered accordingly, in particular based on this combination.

A further step of the method may be the step of visualizing the volume of tissue of a patient to be addressed by the therapy delivery means, especially in the context of the pre-operative and/or peri-operative images of the patient.

Patient images may be loaded and/or displayed a target volume of tissue of a patient may be indicated, especially via voxel painting and/or by free-form drawing of 3D volumes on the patient images.

Further, from the indicated target volume of tissue of a patient the most optimal settings and/or implantation conditions of therapy delivery means may be identified and especially set accordingly by the steering means.

At least one or more effective shape of the volume of tissue (TV) may be displayed and provide a feed-forward simulation of the shape of the volume of tissue (TV) may be provided that shall be provided by the at least one therapy delivery means and patient images may be combined with a feed-forward simulation of the shape of the volume of tissue (TV) that shall be provided by the at least one therapy delivery means.

Information about tissue anisotropy and/or tissue inhomogenity may be used for the simulation when providing a feed-forward simulation of the shape of the volume of tissue (TV) that shall be provided by the at least one therapy delivery means.

Further, the user may identify a target area and may outline a desired shape of the volume of tissue that shall be influenced by the at least one therapy delivery means, wherein especially the desired shape of the volume of tissue that shall be influenced by the at least one therapy delivery means is a desired electrical field coverage volume.

Additionally, at least one boundary-condition may be input and the boundary condition can be a certain range of lead-implantation angles and/or a lead position relative to the target volume (TV) (e.g. lateral to the TV) and/or a certain area to remain unaffected by stimulation.

All steps of the method may be conducted semi-automatically and/or automatically.

It is possible that the above described method and the preferred embodiments thereto for planning and/or providing a therapy for neural applications are only used in-vitro or in-silicio (computer implementation, e.g. testing on a computer only) or for testing and planning purposes only.

However, also it is also explicitly disclosed that the above described method and the preferred embodiments thereto for planning and/or providing a therapy for neural applications may be used for planning and/or providing a therapy for neural applications, especially a neurostimulation and/or neurorecording applications like DBS when the lead is implanted into the patient during therapy.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe of a neurostimulation system for deep brain stimulation (DBS) and its components;
- Fig. 3:: a schematical drawing of a probe system according to the present invention;
- Fig. 4:: a possible embodiment of the system 10 according to the invention; and
- Fig. 5a-c:: step a)-c) how the planning is conducted.

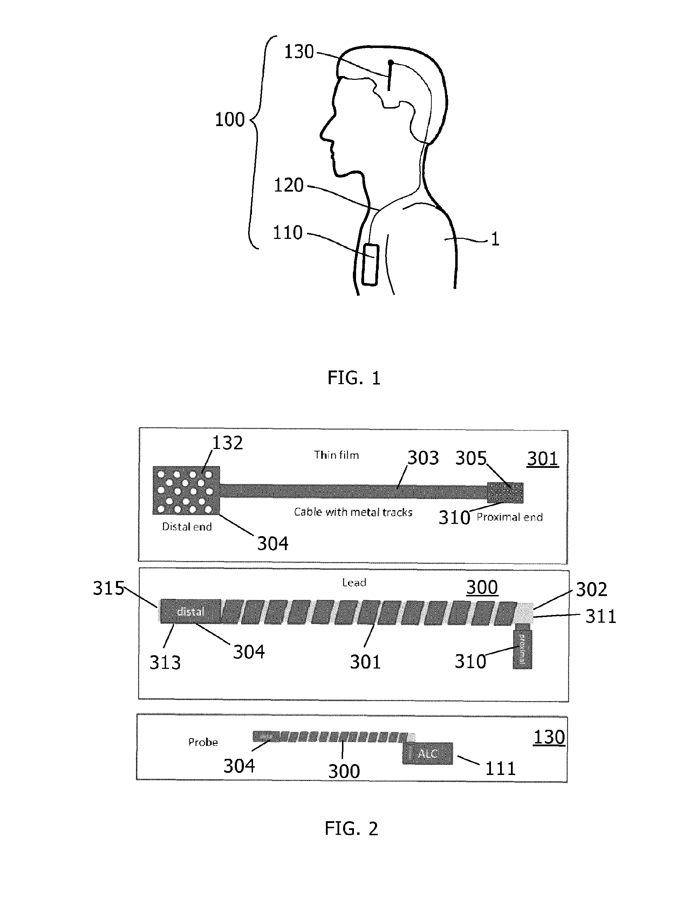

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary electric pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the Advanced Lead Can element 111. The Advanced Lead Can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

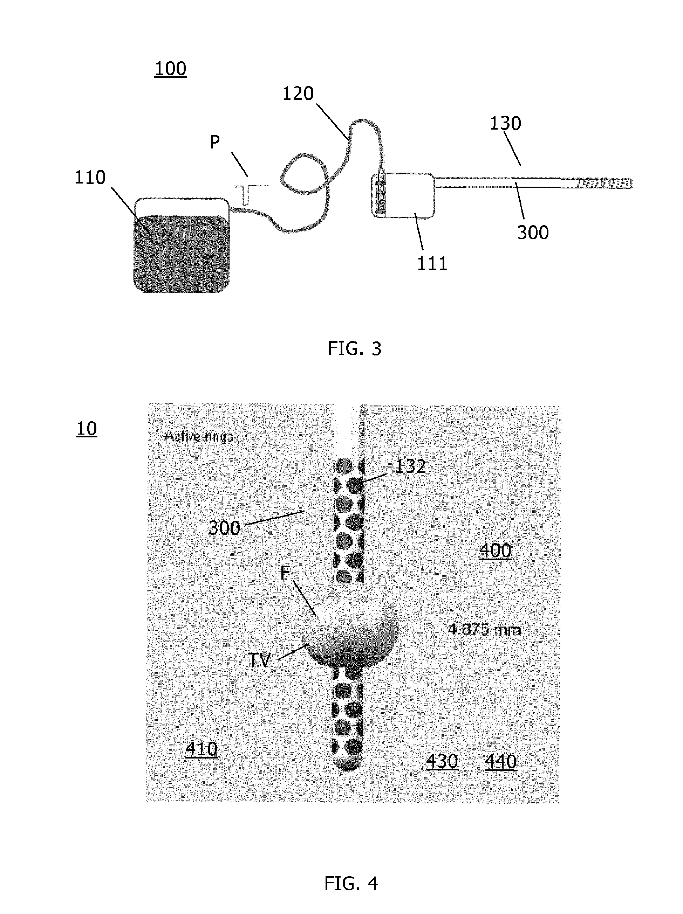

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, wherein e.g. 64 electrodes 132 can be provided on outer body surface at the distal end of the probe 130. By means of the extension wire 120 pulses P supplied by controller 110 can be transmitted to the Advanced Lead Can 111. The controller 110 can be an implantable pulse generator (IPG) 110.

Figure 4 shows a system 10 for planning and/or providing a therapy for neural applications, here for neurostimulation and/or neurorecording applications, i.e. for DBS.

The system 10 comprises at least one therapy delivery means 300, here a DBS lead 300 with a complex array of electrodes 132 being capable to influence a volume of tissue of a patient (1) (see e.g. Figure 1) with a stimulation field F.

The shape of the volume of tissue TV that shall be influenced by the lead 300 may be modelled and the necessary settings and/or implantation conditions of lead 300 may be set accordingly and automatically.

Further, the system 10 comprises at least one processing means 410 being capable to combine specific anatomical data and/or functional data and information about the volume of tissue TV of a patient 1 that can be influenced by the lead 300, wherein the at least one steering and/or field shaping means 400 is configured such that the therapy delivered by the lead 300 may be steered depending on at least one output of the processing means 410.

A touch screen 430 is provided as visualization means 430, which is capable to visualize volume of tissue TV of a patient 1 to be addressed by the lead 300, especially in the context of the pre-operative and/or peri-operative images of the patient 1.

The visualization means 430 is configured such that at least one or more effective shape of the volume of tissue TV may be displayed and that the system 10 comprises a simulation means 440 which is capable to provide a feed-forward simulation of the shape of the volume of tissue TV that shall be provided by the at least one therapy delivery means 300 and that visualization means 430 is configured such that patient images may be combined with a feed-forward simulation of the shape of the volume of tissue TV that shall be provided by the at least one therapy delivery means 300.

The simulation means 440 is configured such that information about tissue anisotropy and/or tissue inhomogenity is used for the simulation when providing a feed-forward simulation of the shape of the volume of tissue TV that shall be provided by the at least one therapy delivery means 300.

Images of the patient 1 are loaded and the user can indicate a target volume TV, e.g. by voxel painting or by free-form drawing of 3D volumes on the patient images. The software as identifying means identifies from the indicated target volume TV a most optimal lead that generates field-shapes overlapping with the user indicated target volume TV. The selected lead is positioned automatically by the system 10 in an orientation that matches the target volume TV, or it can be placed manually by the user on the images and the target volume TV.

Alternatively and/or additionally it is possible that patient images (MRI/CT) are combined with a feed-forward simulation of electrical field(s) F than can be produced by the to-be-implanted DBS system 100. The user selects a DBS lead 300, and for this lead several "effective" volumes can be displayed (non-polarized electric field, directionally dependent electric field, neuronal activation). Volumes can be displayed in 2D by colour coding of MRI voxels (e.g. spread of electric field indicated by red voxels), or using 3D meshes. The user can position the lead 300 and its effective volumes in such a way as to give an optimal result, i.e. good overlap with the target volume TV.

Further alternatively and/or additionally it is possible that information about tissue anisotropy/inhomogenity (deduced from e.g. MRI/DTI) is taken into account in the simulation of the electric field model.

Further alternatively and/or additionally it is possible that the user identifies target area and outlines desired electrical field coverage volume. The system computes and identifies optimal lead for this and shows the lead 300 and stimulation capabilities on top of this.

Also, it is possible to extend the capabilities of the system 10 by allowing the user to provide more boundary-conditions, e.g. by demanding a certain range of lead-implantation angles, by demanding a lead position relative to the TV (e.g. lateral to the TV), by demanding certain areas to remain unaffected by stimulation, etc. Essentially, that will narrow the search-space for lead types and may narrow the orientation/location of the lead with respect to the target volume TV.

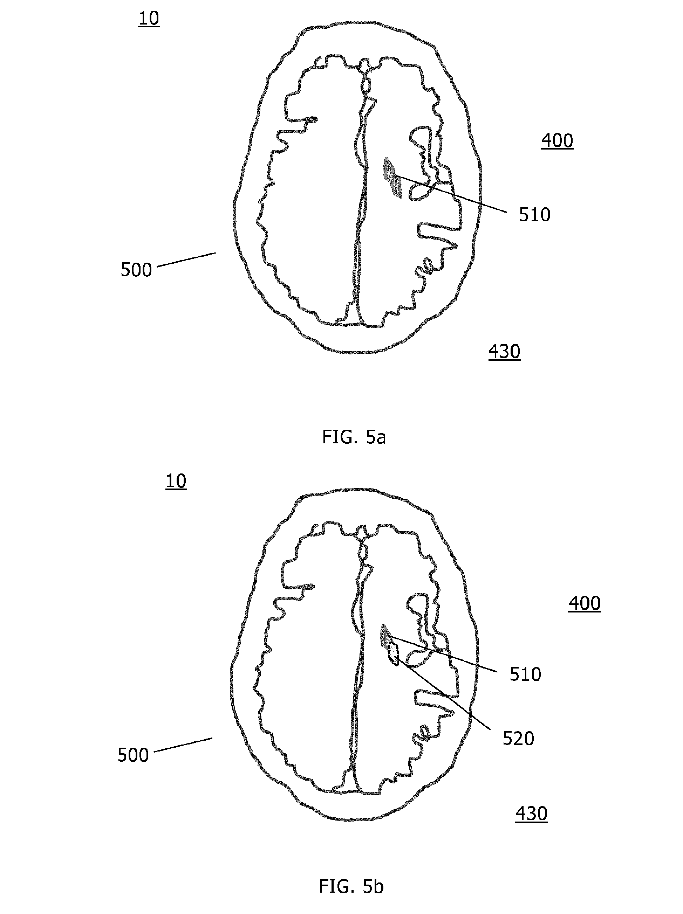

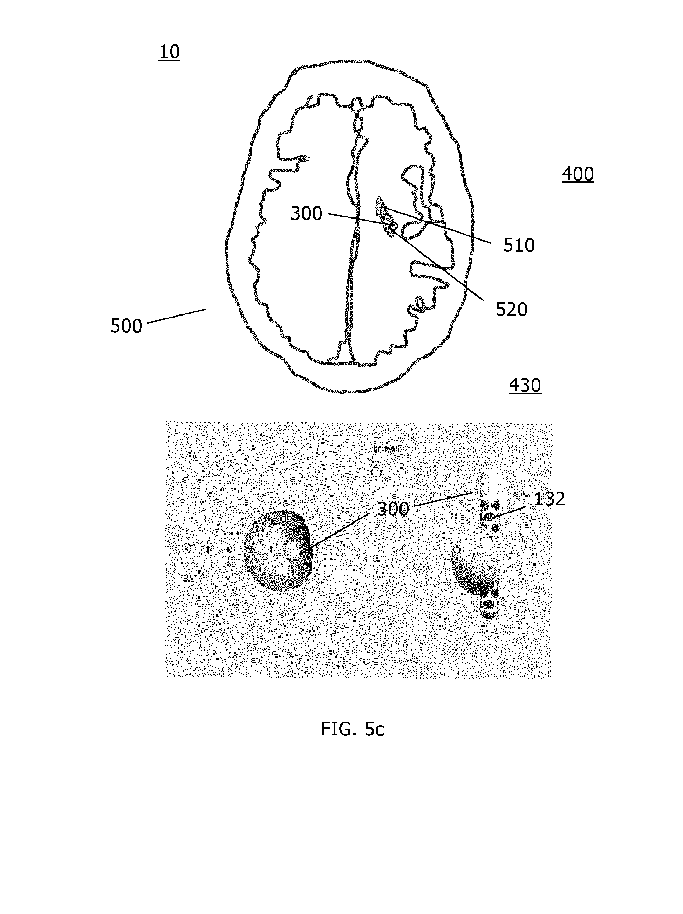

Figures 5a, 5b and 5c show a possible workflow for the method for planning and/or providing a therapy for neural applications, especially a neurostimulation and/or neurorecording applications in the field of Deep Brain Stimulation may be conducted, comprising at least the shown three steps:

In a first step as shown in Figure 5a a medical image 500 is provided and visualized by the visualization means 430. Within this medical image 500 a relevant brain region 510 is marked in a first manner and/or colour (e.g. in red). This can be done by means of the input means (not shown) of the system 10.

In a second step as shown in Figure 5b a physician specifies the intended therapy coverage area 520 in a second manner and/or colour (e.g. in yellow).

Then, in a third step the system 10 as shown in Figure 5c suggests the therapy delivery means 300 with the required therapy parameter settings (lower panel), the implantation location of this therapy delivery means 300 (upper panel, dot - here grey dot) and the resulting volume of tissue where therapy will be delivered.

## Claims

1. A system (10) for planning and/or providing a therapy for neural applications, especially for neurostimulation and/or neurorecording applications, comprising and/oder being connected and/or being connectable to at least one therapy delivery means (300) being capable to influence a volume of tissue of a patient (1), further comprising a steering and/or field shaping means (400) which is configured such that at least the shape of the volume of tissue (TV) that shall be influenced by the at least one therapy delivery means (300) may be modelled and the necessary settings and/or implantation conditions of therapy delivery means (300) may be set accordingly.

2. The system (10) according to claim 1,
**characterized in that**
the system (10) comprises at least one processing means (410) being capable to combine specific anatomical data and/or functional data and information about the volume of tissue (TV) of a patient (1) that can be influenced by the at least one therapy delivery means (300), wherein the at least one steering and/or field shaping means (400) is configured such that the therapy delivered by the therapy delivery means (300) may be steered depending on at least one output of the processing means (410).

3. The system (10) according to claim 1 or 2,
**characterized in that**
the therapy delivery means (300) is or comprises a drug delivery system and/or a system for delivery of ultrasound energy like an array of transducers for delivery of ultrasound energy and/or a light-emitting system like an array of light-conductors, in particular e.g. waveguides.

4. The system (10) according to one of the preceding claims, in particular according to claim 1 or 2,
**characterized in that**
the therapy delivery means (300) is or comprises a lead (300) for neural applications, especially for neurostimulation and/or neurorecording applications.

5. The system (10) according to claim 4,
**characterized in that**
the lead (300) comprises a plurality of electrodes (132), wherein the lead (300) is capable to provide and/or generate at least one stimulation field (F), wherein especially the plurality of electrodes (132) forms a complex geometrical array and/or that the plurality of electrodes (132) is arranged circumferentially around at least one section of the lead (300), especially around a section next to the distal tip end of the lead (300).

6. The system (10) according to one of the preceding claims, **characterized in that**
the system (10) comprises a visualization means (430), wherein the visualization means (430) is capable to visualize volume of tissue (TV) of a patient (1) to be addressed by the therapy delivery means (300), especially in the context of the pre-operative and/or peri-operative images of the patient (1).

7. The system (10) according to claim 6,
**characterized in that**
the visualization means (430) comprises input means and output means and is configured such that patient images may be loaded and/or displayed by means of the output means and that by means of the input means a target volume of tissue (TV) of a patient (1) may be indicated, especially via voxel painting and/or by free-form drawing of 3D volumes on the patient images.

8. The system (10) according to claim 7,
**characterized in that**
the system comprises an identifying means which is configured such that from the indicated target volume of tissue (TV) of a patient (1) the most optimal settings and/or implantation conditions of therapy delivery means (300) may be identified and especially set accordingly by the steering means (400).

9. The system (10) according to one of claims 6 to 8, **characterized in that**
the visualization means (430) is configured such that at least one or more effective shape of the volume of tissue (TV) may be displayed and that the system (10) comprises a simulation means (440) which is capable to provide a feed-forward simulation of the shape of the volume of tissue (TV) that shall be provided by the at least one therapy delivery means (300) and that visualization means (430) is configured such that patient images may be combined with a feed-forward simulation of the shape of the volume of tissue (TV) that shall be provided by the at least one therapy delivery means (300).

10. The system (10) according to claim 9,
**characterized in that**
the simulation means (440) is configured such that information about tissue anisotropy and/or tissue inhomogenity is used for the simulation when providing a feed-forward simulation of the shape of the volume of tissue (TV) that shall be provided by the at least one therapy delivery means (300).

11. The system (10) according to one of claims 6 to 10, **characterized in that**
the visualization means (430) is configured such that the user may identify a target area and may outline a desired shape of the volume of tissue (TV) that shall be influenced by the at least one therapy delivery means (300), wherein especially the desired shape of the volume of tissue (TV) that shall be influenced by the at least one therapy delivery means (300) is a desired electrical field coverage volume.

12. The system (10) according to one of the preceding claims, **characterized in that**
the steering and/or field shaping means (400) is configured such that at least one boundary-condition may be input, wherein especially the boundary-condition may be input via the input means.

13. The system (10) according to claim 12,
**characterized in that**
the boundary condition is a certain range of lead-implantation angles and/or a lead position relative to the target volume (TV) (e.g. lateral to the TV) and/or a certain area to remain unaffected by stimulation.

14. The system (10) according to one of the preceding claims, **characterized in that**
the steering and/or field shaping means (400) is configured such that at least the shape of the volume of tissue (TV) that shall be influenced by the at least one therapy delivery means (300) may be modelled and the necessary settings and/or implantation conditions of therapy delivery means (300) may be set accordingly semi-automatically and/or automatically.

15. A system for neural applications (100), especially a system for neurostimulation and/or neurorecording applications (100), for instance a deep brain stimulation system (100), the system for neural applications (100) comprising at least one system (10) for planning and/or providing a therapy for neural applications according to one of the preceding claims.
